# EUROPEAN PATENT APPLICATION

(11) **EP 4 588 470 A1**
(43) Date of publication of application: **23.07.2025**
(21) Application number: 24153009.6
(22) Date of filing: 19.01.2024
(51) Int. Cl.: A61K 9/50, A61K 31/05, A61K 36/324

(54) **PHARMACEUTICAL COMPOSITION COMPRISING CURCUMIN AND THE PREPARATION THEREOF**

(71) Applicant: Löffler, Bernd-Michael, 10717 Berlin (DE)
(72) Inventor: The designation of the inventor has not yet been filed
(74) Representative: Braeuning Schubert Patentanwälte GbR

(57) **Abstract**

The object of the present invention is a pharmaceutical composition, comprising a combination preparation comprising a first active ingredient derived from a plant oleoresin from plants of genus curcuma and at least one second active ingredient, double micro-encapsulated by a layer of carbohydrate polymers and lipids, wherein the lipids are derived from a plant oleoresin A further object of the present invention is a method for the production of a double micro-encapsulated pharmaceutical composition according to the invention and a pharmaceutical drug comprising said pharmaceutical composition according to the invention.

## Description

### Technical Field

The present invention relates to a novel pharmaceutical composition containing at least curcumin, wherein the bioavailability of curcumin is very high and wherein the composition comprises further different curcuminoids. The present invention relates also to a novel method for the preparation of the inventive pharmaceutical composition as described above.

### Background Art

For thousands of years, natural products have been used in traditional medicines, and they have shown promise as a source of components for the development of new drugs. Curcumin (1,7-bis(4-hydroxy-3-methoxyphenyl)-1,6-heptadiene-3,5-dione), also known as diferuloylmethane, is a crystalline compound that has a bright orange-yellow color and the primary natural polyphenol found in Curcuma longa rhizome (turmeric). The aroma of this spice is principally derived from α- and β-turmerone and aromatic turmerone.

Curcuminoids have attracted potential therapeutic interest for the treatment of immune-related, metabolic, and cancer-related diseases due to their wide range of biological targets and lack of side effects. Curcumin has recently gained recognition as an important natural intervention due to its wide range of biological activities, including antimicrobial, anticancer, anti-inflammatory, antitumor, antioxidant, radioprotective, cardioprotective, and neuroprotective properties, despite its very low solubility, poor absorption, bioavailability, rapid elimination, and metabolic changes.

Curcumin works via different physiological mechanisms, for instance curcumin acts as an anti-inflammatory by reducing ROS (reactive oxygen species) production due to its effect on nicotinamide adenine dinucleotide phosphate (NADPH) oxidase and increasing the activity of antioxidant enzymes. Also Curcumin has been shown in animal studies to increase the survival of tumor-bearing rodents by inhibiting tumor growth and preventing metastasis.

There is a growing interest in using phytochemicals to prevent prostate cancer, as well as to treat oxidative and inflammatory conditions, metabolic syndrome, arthritis, anxiety, and hyperlipidemia. Management of exercise-induced inflammation and muscle soreness, thereby improving recovery and subsequent performance in active people, etc. led us to investigate potential preventive agents such as curcumin.

However, concerns have been raised about its low bioavailability and poor absorption in the gastrointestinal tract, limiting its clinical use. Curcuminoids are naturally amphiphilic and they are more soluble in organic solvents than in water, and curcumin's solubility in aqueous solution is less than 0.03 M in buffer at pH 7. As a result, curcumin has poor gastrointestinal absorption and low aqueous solubility. Several animal studies have shown that the majority of oral curcumin (90%) is excreted in the faeces. To address this issue, numerous methods for increasing bioavailability have been developed. According to recent research, it has been found that Curcumin encapsulation may improve systemic bioavailability.

These include the use of adjuvants like piperine, the formulation of liposomal curcumin, curcumin nanoparticles, curcumin phospholipid complexes, and the use of curcumin structural analogues like turmeric oil.

In the state of the art WO 2015/025236 A1 discloses a curcumin composition for increasing the bioavailability of curcumin, which consists of curcumin mixture and water extract in a ratio of 70:30. The curcumin mixture comprises curcumin dry crystals, volatile oil, fixed oil whereas water extract comprises soluble proteins, dietary fibers and carbohydrates extracted from turmeric. The composition also consists of a natural emulsifier isolated from *Quillaja Saponaria* and lecithin. The composition increases rate of availability of curcumin as the dietary fibers holds the curcumin for long time. The soluble proteins increase the solubility of curcumin in water thus increasing absorption of curcumin in blood and the essential oil increases dispensability. The curcumin composition showed bioequivalence with 250 mg curcumin capsules, exhibited anti-aging, antioxidant, anti-inflammatory activities and is also effective in boosting the phagocyte mediated immunity suggesting as natural therapy for treatment of various diseases.

Further, WO 2022/219166 A1 discloses a combination preparation comprising resveratrol and at least one gingerol, or resveratrol and a combination of gingerols, in addition to pharmaceutical or nutraceutical acceptable adjuvants and additives.

### Summary of the Invention

Therefore, it is the problem of the invention to overcome the disadvantages of the state of the art.

The problem of the invention is solved by providing a pharmaceutical composition, comprising a combination preparation comprising a first active ingredient derived from a plant oleoresin from plants of genus *curcuma* and at least one second active ingredient, double micro-encapsulated by a layer of carbohydrate polymers and lipids, wherein the lipids are derived from a plant oleoresin. Different experimental set-ups as detailed in the following description have shown that the bioavailability of curcumin is enhanced when using a pharmaceutical composition according to the present invention. The synergic effect of curcumin covered by a turmeric lipid encapsulated in a wall material such as modified food starch can significantly enhance the bioavailability even further. When using turmeric oleoresin as the base of lipid layer, no other extra materials are required. Furthermore turmeric oleoresin contain turmeric essential oil and curcuminoid and both have a hypoglycemic effect.

In a preferred embodiment of the present invention a pharmaceutical composition is provided, wherein at least the second active ingredient is resveratrol, gingerol or is derived from oleoresin from plants of genus *Boswellia.* Furthermore preferred is a pharmaceutical composition according to the present invention, wherein the combination preparation comprises a third active ingredient, which is selected from resveratrol, gingerol or is derived from a plant oleoresin from plants of genus *Boswellia.*

Adding a second or even third active ingredient leads to a synergistic effect. For example, there are functional overlaps between curcumin, resveratrol and gingerol. In addition to its own highly interesting effects, gingerol especially 6-gingerol is an "amplifier" of curcumin and resveratrol. Furthermore curcumin and resveratrol also have signaling in common of the cell, which they can both activate or inhibit simultaneously in the same direction.

Further preferred is a pharmaceutical composition, according to the present invention, wherein the lipids comprise a selection and/or a combination of fatty acids, glycerolipids, glycerophospholipids, sterols, prenols, saccharolipids and polyketides.

Additionally preferred is a pharmaceutical composition, according to the present invention, wherein the carbohydrate polymers are selected from one or more of the group comprising starch, modified starch, poly anhydrides, poly (ortho esters), poly (ethylene carbonates), poly (lactic acids), poly (glycol acids), poly (D,L-lactide-co-glycolide) and co-poly (L-lactic acid-oxyethylene-β-L-lactic acid).

Furthermore preferred is a pharmaceutical composition, according the present invention, wherein the first active ingredient is derived from plant oleoresin of genus *curcuma* comprises curcuminoids, especially curcumin, demethoxycurcumin, bis-demethoxycurcumin.

Further preferred is a pharmaceutical composition, according to the present invention, wherein the at least one second or third active ingredient is derived from plant oleoresin of genus *Boswellia* comprises α-boswellic acid, acetyl-α-boswellic acid, acetyl-11-*keto*-α-boswellic acid, β-boswellic acid, acetyl-β-boswellic acid, 11-*keto*-β-boswellic acid (KBA), and/or acetyl-11-*keto*-β-boswellic acid (AKBA).

Additionally preferred is the pharmaceutical composition, according to present invention, wherein the at least one second or third active ingredient of gingerol further comprises shogaols, which are selected from 4-shogaol, 6-shogaol, 8-shogaol, 10-shogaol and 12-shogaol.

Preferred is a pharmaceutical composition, according to the present invention, wherein the double micro-encapsulated layer consists of an inner and an outer layer.

Especially preferred is a pharmaceutical composition, according to the present invention, wherein the inner layer comprises a homogenized and/or complex mixture of a combination of the first active compound, namely a derivative from a plant oleoresin from plants of genus curcuma and a second active compound, namely resveratrol, gingerol or a derivative from plant oleoresin from plants of genus *Boswellia.*

Also preferred is a pharmaceutical composition, according to the present invention, wherein the outer layer comprises a homogenized and/or complex mixture of a combination of the first active compound, namely a derivative from a plant oleoresin from plants of genus curcuma and a second active compound, namely resveratrol, gingerol or a derivative from plant oleoresin from plants of genus *Boswellia.*

To improve bioavailability even further a double shielding of curcumin is used within a lipid layer of turmeric oil and a suitable wall material as the outer covering.

Furthermore, the problem of the present invention is solved by providing a method for the production of a double micro-encapsulated pharmaceutical composition according to the present invention, successively comprising the steps of a. providing a plant oleoresin; b. separating active ingredients in form of crystals from the plant oleoresin and separating lipids in form of liquids from the plant oleoresin; c. mixing of the separated crystals and the separated lipids as obtained in step b. with carbohydrate polymers and forming an aqueous solution; d. homogenization of the aqueous solution produced in step c.; e. spray drying of in step d. produced homogenized aqueous solution and producing a double micro-encapsulated pharmaceutical composition. Due to the method according to the invention, the preparation is improved and thus the bioavailability of curcumin is increased. The micro-encapsulation process protects the pharmaceutical composition from harmful environmental conditions. It utilizes wall materials such as starch for protecting the nucleus-core material.

An embodiment of the method according to the present invention is preferred, wherein the providing of plant oleoresin according to step a. is performed in that a rhizome of the utilized plant of interest is dried and crushed and in that the oleoresin is extracted from the crushed rhizome material.

Furthermore preferred is a method according to the present invention, wherein the extraction is performed using organic solvents comprising alcohols, like ethanol, methanol, and the like and/or acetone and/or hydrocarbons, like n-hexane, iso-hexane, n-pentane, iso-pentane, and the like and/or steam of water and/or supercritical fluid extraction using supercritical carbon dioxide.

Additionally preferred is a method according to the present invention, wherein the providing of plant oleoresin according to step a. is performed in that the oleoresin is extracted from excretion cells of the bark, especially from the plants of genus *Boswellia.*

Further preferred is a method according to the present invention, wherein in an additional step f. after step e. the product retrieved in step e., namely the double micro-encapsulated pharmaceutical composition is mixed with pharmaceutical additives or adjuvants and formed into a tablet or a capsule, wherein preferably the tablet or the capsule is an enteric coated tablet or an enteric coated capsule.

Further, the problem of the present invention is solved by a pharmaceutical drug in form of an tablet or capsule, comprising a pharmaceutical composition according to the present invention.

"Comprising" means in that context that further pharmaceutical drug components or other components can be present in the pharmaceutical drug. However, it does not exclude that no further components beside the pharmaceutical composition according to the invention are present in the pharmaceutical drug.

### Brief Description of Drawings

Fig. 1 shows a double micro-encapsulated product according to the present invention;
Fig. 2 shows a flow chart of the method for the production of a pharmaceutical product according to the present invention;
Fig. 3 shows a graphical representation of serum concentrations of total curcuminoids in rats;
Fig. 4 shows a graphical representation of serum concentrations of desmethoxycurcumin in rats;
Fig. 5 shows a graphical representation of serum concentrations of bisdesmethoxycurcumin in rats;
Fig. 6a and Fig. 6b shows graphical representations of comparing pharmacokinetic of CUREmin-ACTIF^{®} and a pharmaceutical composition according to the present invention;
Fig. 7 shows a graph of mean AUC's of Curcuminoid, CUREmin-ACTIF^{®} and the pharmaceutical composition according to the present invention;
Fig. 8 shows a graph depicting the inhibitory effect of conventional chemotherapeutics;
Fig. 9 shows a graph depicting the inhibitory effect of Resveratrol-ACTIF^{®} and the pharmaceutical composition according to the present invention;
Fig. 10 shows a graph depicting the results for chemotherapeutics and Resveratrol-ACTIF^{®} and the pharmaceutical composition according to the present invention.

### Detailed Description of the Invention

The matter of the present invention shall be made clear and concise with the following definitions for specific terms used in the present description.

The term *"crystallized curcuma extract"* means that further pharmaceutically active substances like curcuminoids, especially curcumin, demethoxycurcumin and bis-demethoxycurcumin, as well as to their isomeric forms, especially as keto-enol tautomeric forms, and gingerols, selected from shogaols, especially 4-shogaol, 6-shogaol, 8-shogaol, 10-shogaol, and 12-shogaol as well as gingerols, especially 6-gingerol, 8-gingerol, and 10-gingerol which are compromised after the extraction and crystallization.

The term *"turmeric lipid"* means that lipids which are insoluble in water have been extracted from the turmeric rhizome. It is known in the state of the art that lipids are fatty acids, glycerolipids, glycerophospholipids, sterols, prenols, saccharolipids, polyketides and the like. The turmeric lipids can be used for micro-encapsulation of pharmaceutical compounds.

The term *"carbohydrate polymer substance"* means natural or synthetic carbohydrates like starch, modified starch, poly anhydrides, poly (ortho esters), poly (ethylene carbonates), poly (lactic acids), poly (glycol acids), poly (D,L-lactide-co-glycolide), co-poly (L-lactic acid-oxyethylene-β-L-lactic acid) and the like. Modified starch is the main product used for micro-encapsulation as known in the state of the art. The person skilled in the art knows all suitable substances for the preparation of micro-encapsulation.

The term *"oleoresin"* describes semi-solid extracts composed of resin and essential or fatty oil.

The term "Boswellia" describes specially AKBA (3-O-acetyl-11-keto-β-boswellic acid) the most active molecule of boswellia resins, which can be extracted from resins of *Boswellia serrata* and *Bossewellia carteri.*

The term "resveratrol" describes 3, 4', 5 trihydroxystilbene, which is a naturally occurring phytoalexin produced by some spermatophytes, such as grapevines, in response to injury .It is produced by plant sources such as grapes, apples, blueberries, plums, and peanut. Further resveratrol can be encapsulated within an oil part of other plants of ginger kind of species and can be microencapsulated by using a starch solution.

The term "gingerol" describes components of ginger plants, that are members of the ginger family, which are broadly used as dietary supplements or bioactive compound for different disease.

The term "pharmaceutical product" or "pharmaceutical drug" describes any known forms such as tablets, sucking tablets, film tablet, dragees, capsules or pills, which is made of the pharmaceutical composition according to the invention.

The term "Test Sample" describes a pharmaceutical composition according to the invention, which has been produced by using the method according to the invention.

### Pharmaceutical Composition

In general, usual pharmaceutical preparations can be produced according to the state of the art.

A preferred subject of the present invention is a pharmaceutical composition for oral, buccal, sublingual, nasal, rectal, subcutaneous, intravenous or intramuscular application as well as for inhalation, which, in addition to the usual vehicle and dilution agents, also contains the active ingredients according to the present invention. The pharmaceutical composition according to the present invention is produced in the known way and in suitable dosage, with the usual solid or liquid vehicle substances or dilution agents and the usually used pharmaceutical-technical adjuvants corresponding to the desired type of application. The inventive compositions are present in a form of administration which is suitable for oral application.

Such forms of administration include, for example, tablets, sucking tablets, film tablets, dragees, capsules, pills, powders, solutions, aerosols or suspensions or slow-release forms.

Corresponding tablets can be obtained, for example, by mixing the active substance with known adjuvants, for example, inert dilution agents such as dextrose, sugar, sorbitol, mannitol, polyvinylpyrrolidone, bursting agents such as corn starch or alginic acid, binders such as starches or gelatins, lubricants such as magnesium stearate or tale and/or agents for achieving a slow release effect such as carboxypolymethylene, carboxymethylcellulose, cellulose acetate phthalate or polyvinyl acetate. The tablets may consist of several layers.

Dragees can also be produced correspondingly, for controlled or delayed release forms of preparation, by coating the cores produced analogously to the tablets with agents commonly used in dragee coatings, for example, polyvinylpyrrolidone or shellac, gum arabic, talc, titanium dioxide or sugar. The dragee envelope may also consist of several layers, wherein the adjuvants mentioned above in the case of tablets can be used.

Solutions or suspensions containing the active ingredients used according to the invention may additionally contain agents that improve taste, such as saccharin, cyclamate or sugar, as well as, e.g., taste enhancers such as vanilla or orange extract. They may also contain suspension adjuvants such as sodium carboxymethylcellulose or preservatives such as p-hydroxybenzoate.

Capsules containing active substances can be produced, for example, by mixing the active ingredients with an inert vehicle such as lactose or sorbitol and encapsulating this mixture in gelatin capsules. Suitable suppositories can be produced, for example, by mixing with vehicle agents provided therefore, such as neutral fats or polyethylene glycol or derivatives thereof.

The production of the pharmaceutical preparations or compositions according to the invention is known in and of itself, and is described in handbooks known to the person skilled in the art, for example, Hager's Handbuch [Handbook] (5th ed.) 2, 622-1045; List et al., Arzneiformenlehre [Instructions for Drug Forms], Stuttgart: Wiss. Verlagsges. 1985; Sucker et al., Pharmazeutische Technologie [Pharmaceutical Technology], Stuttgart: Thieme 25 1991; Ullmann's Enzyklopädie [Encyclopedia] (5th ed.)A 19, 241-271; Voigt, Pharmazeutische Technologie [Pharmaceutical Technology], Berlin: Ulistein Mosby 1995.

### Description of Embodiments

Curcumin, the active ingredient is wrapped inside a turmeric lipid layer. By using the turmeric oleoresin as the base of lipid layer, no other extra materials are required and can enhance the bioavailability notably. Also turmeric oleoresin contain turmeric essential oil and curcuminoid and both having hypoglycemic effect. Hence makes the best choice for making the lipid layer around. That is multipurpose is in action. Due to the lipophilic nature, it can cross the lipid bilayer and can noticeably use for a variety of applications. The essential oils are composed mainly of sesquiterpenes, many of which are specific for the Curcuma genus. The lipid wrapped curcumin is then loaded into a matrix such as starch. The effective matrix system could load and protect curcuminoids, then deliver them at the target. Starch is a wall material that is commonly used in micro-encapsulation. Modified Starch, are increasingly being considered for micro-encapsulation of vitamins, essential oils, flavors and phytochemicals. Micro-encapsulation is a process that protects compounds from harmful environmental conditions. It utilizes wall materials such as starch for protecting the nucleus-core material. This wall material act as a protecting cover from external stimuli such as pH, light, humidity etc. Starch can be modified chemically, physically, or enzymatically to improve or change its functionality in order to produce higher yielding micro-encapsulation of compounds while retaining the functionality and physicochemical properties of the nucleus, which should still be easily released. Changes in pH, temperature, diffusion by external fluid action, chemical reactions, enzymatic hydrolysis, or mechanical rupture cause the core material to be released from the encapsulating material. Thus in total, the double micro-encapsulation technique successfully enhance the effect of the product.

It is known in the state of the art that curcumin, based on *Curcuma longa,* usually consists of about 77 % curcumin I (curcumin), about 17 % curcumin II (demethoxycurcumin) and about 3 % curcumin III (bis-demethoxycurcumin). Furthermore, Curcuma longa, usually comprises shogaols, like 4-shogaol, 6-shogaol, 8-shogaol, 10-shogaol, and 12-shogaol, gingerols, like 6-gingerol, 8-gingerol, and 10-gingerol as well as turmeric lipids. All these compounds can be extracted from a turmeric rhizome or respective plant materials from dried rhizomes of ginger and are also used according to the present invention.

It has to be pointed out that according to the invention other rhizomes from the family of *Zingiberaceae* and/or with the genus *Curcuma* can be used for the pharmaceutical composition according to the present invention. It is also clear that these different rhizome can be used to be performed according to the production of the pharmaceutical compound as described herein.

### Example 1

### Characteristic details of double micro-encapsulated inventive pharmaceutical product comprising curcuma extract

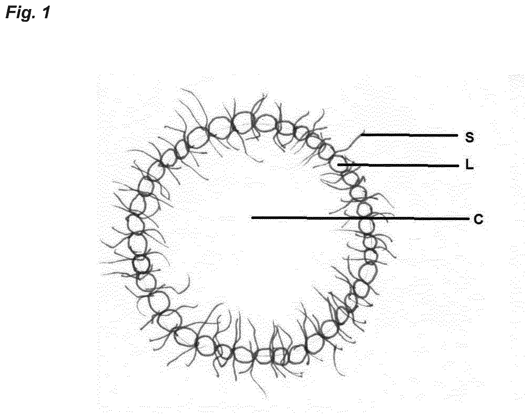

Figure 1 illustrates in a cross-sectional view the arrangement of the active ingredient curcuma extract C and the encapsulants turmeric lipid L and starch S for the double micro-encapsulation. Curcuma extract C is completely enclosed with the turmeric lipid L. The turmeric lipid L is in form of a great amount of single spheres which encapsulate curcumin C. The second or double encapsulation is performed with starch S, wherein the starch molecules partially penetrate the turmeric lipid spheres. This means that the starch molecules S contact the inner curcuma extract C and the outer surface created with the turmeric lipid L of the final product. This explanation discloses the double micro-encapsulation.

### Example 2

Details about production of double micro-encapsulation of curcuma extract

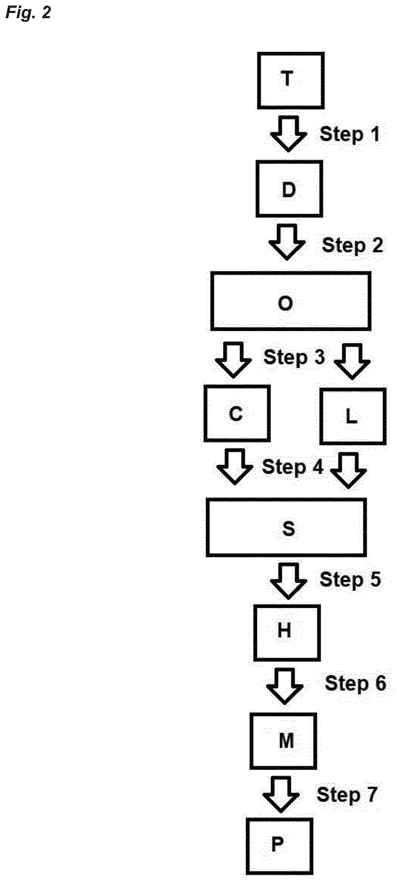

Figure 2 illustrates the inventive method of production of a double micro-encapsulated pharmaceutical product. As illustrated in Figure 2 the process of the production is performed as described in seven steps.

The starting material is turmeric T, a rhizome of *curcuma longa,* which is a part of the family of *Zingiberaceae.* It is also possible to use other rhizomes of the family of *Zingiberaceae* or the genus of *curcuma.* In **Step 1,** the turmeric is dried and dried turmeric D is used in following Step 2. **Step 2** extracts active ingredients from turmeric T in form of oleoresin O. The extraction is performed in a usual manner as known in the state of the art. The extraction is performed via organic solvents comprising alcohols, like ethanol, methanol, and the like and/or acetone and/or hydrocarbons, like n-hexane, iso-hexane, n-pentane, iso-pentane, and the like and/or steam of water and/or supercritical fluid extraction using supercritical carbon dioxide and the like as known in the state of the art. The final product of the extraction process is oleoresin O. The extracted oleoresin from turmeric T or dried turmeric D comprises etheric oils and resins as well as organic compounds. All these extracted substances are useful as pharmaceutical ingredients. These substances may comprise curcuminoids, turmeric lipids, shogaols, like 4-shogaol, 6-shogaol, 8-shogaol, 10-shogaol, and 12-shogaol, gingerols, like 6-gingerol, 8-gingerol, and 10-gingerol as well as turmeric lipids. Turmeric lipids are not water soluble and have therefore been extracted as described above. In **Step 3** the extracted oleoresin O is prepared in order to crystalize curcumin C out of the oleoresin O. After the extraction of curcuma extract C from oleoresin O, turmeric lipids L remain and are also separated. This means that **Step 3** separates crystallized curcumin C and turmeric lipids L from oleoresin O. The products obtained from **Step 3,** namely crystallized curcuma extract C and turmeric lipids L are in **Step 4** now added into a starch solution S and the solution mixture of starch S, crystallized curcuma extract C and turmeric lipids L are then in **Step 5** homogenized in order to obtain a homogenized intermediate product H. In next **Step 6,** a spray drying of the homogenized intermediate product H is performed to obtain a double microencapsulated intermediate product M as described in Figure 1. In final **Step 7** the double micro-encapsulated intermediate product M is prepared as a pharmaceutical product P.

### Example 3

Details about production of double micro-encapsulation of oleoresin extracted from plants of genus *Boswellia*

The inventive production of a pharmaceutical composition according to the present invention comprising active ingredients from plants of genus *Boswellia* can be performed in using the oleoresin extracted from the bark of *Boswellia* plants, especially from *Boswellia serrata* or *Boswellia carteri..*

The starting material is *Boswellia serrata,* which is a part of the family of *Bur-seraceae.* The *Boswellia serrata* oleoresin occurs in transparent, yellow brown fragrant tears, which are about 5 cm long and 2 cm thick, containing 20-36% gum (galacturonic acid, digitoxose, arabinose, galactose and xylose), 56-65% acid resin (up to 43% triterpene acids) and 4-8% essential oil. In a first step Boswellia serrata oleo-gum resin is air-dried and grounded. In the next step the air-dried oleo-gum resin is used to isolate essential oil. The isolation is performed in a usual manner as known in the state of the art. The isolation is performed by either hydro- and steam distillation or supercritical fluid extraction using supercritical carbon dioxide . The final product of the extraction process is the essential oil, which is collected.. In a parallel step the air dried and grounded oleo-gum resin is used to isolate 3-O-acetyl-11-keto-β-boswellic Acid (AKBA). The isolation is performed by hydroalcoholic extraction followed by alkali treatment and further treatment with acid to precipitate the bioactive compound AKBA as known in the state of the art. The extracted products, namely the oil part and acid resin part (AKBA) are added into a starch solution and the solution mixture of starch, oil and AKBA are then homogenized in order to obtain homogenized intermediate product. In the next step, a spray drying of the homogenized intermediate is performed to obtain double microencapsulated product as described in Figure 1. Further the double micro-encapsulated intermediate product is prepared as a pharmaceutical product.

### Example 4

Comparative study to assess single dose oral bioavailability of CUREmin ACTIF^{®} 2 capsule and Test Sample 3, which is the inventive pharmaceutical composition, in rats.

In total three experimental set-ups were designed, whereas in each experimental set-up three treatment groups were determined; each treatment group consisting of 10 rats. In case of all experimental set-ups one group obtained a Control 1, one group obtained CUREmin-ACTIF^{®} 2, and one group obtained the Test Sample 3. Each compound was given in a doses of 250 mg compound-preparation per kg weight by oral gavage. Compounds have been applicated in fasting state.

In all experimental set-ups the Control 1 is pure curcumin (conventional curcumin) or curcumin 95%; CUREmin-ACTIF^{®} 2 is a capsule of 400mg/dose containing 250mg Curcuminoids, 50mg Turmeric Extract and 100mg Sodium Starch octenyl succinate. The Test Sample 3 was in all experimental set-ups a pharmaceutical composition according to the invention. The test sample is a capsule containing 250mg Curcuminoids, 50mg Turmeric Extract and 100mg Sodium Starch octenyl succinate, which was produced by using the method according to the present invention.

The serum concentration of curcumin was determined in all three experimental set-ups to different time-points within a time span of 24 hours after oral uptake.

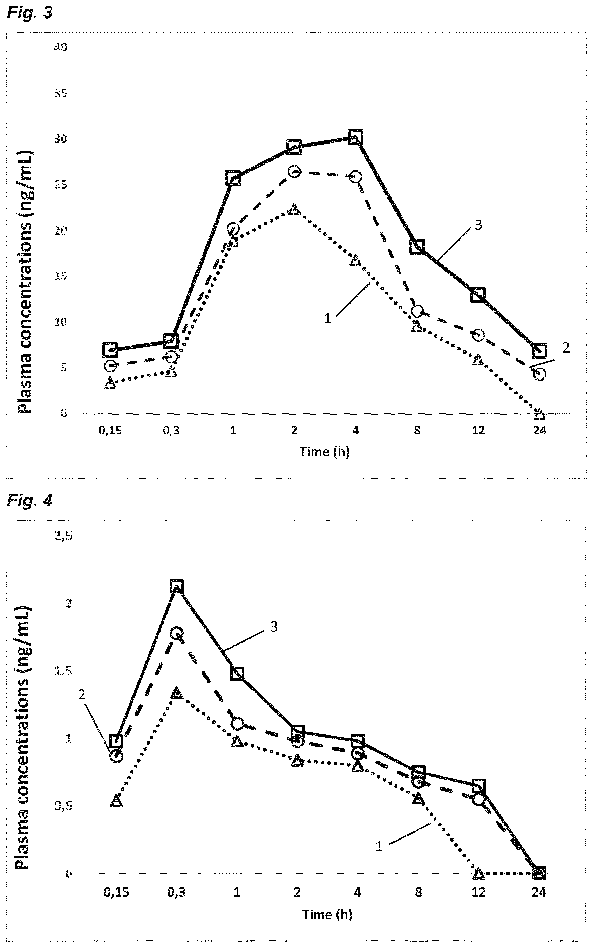

The first experimental set-up was designed to determine serum concentrations of total curcuminoids. The results are summarized in Table 1 and the graphic representation is shown in Figure 3. The pharmaceutical composition according to the invention, namely Test Sample 3 showed a higher serum concentration of curcumin I compared to CUREmin-ACTIF^{®} 2 as well as to the control 1. A peak of curcumin serum concentration was shown to be two to four hours after oral uptake. Those results demonstrate a higher bioavailability of curcumin when provided by using a pharmaceutical composition according to the invention.

**Table I**

| **Time (h)** | **Control (n=10) (ng/mL) Mean +/- SD** | **CUREmin-ACTIF^{®} (n=10) (ng/mL) Mean +/-SD** | **Test Sample (n=10) (ng/mL) Mean +/-SD** |
|---|---|---|---|
| **0** | **0** | **0** | **0** |
| 0,15 | 3,41±0,11 | 5,22±0,09 | 6,91±0,18 |
| 0,3 | 4,62±0,13 | 6,21±0,10 | 7,89±0,21 |
| 1 | 18,97±0,07 | 20,23±0,25 | 25,68±0,16 |
| 2 | 22,46±0,13 | 26,47±0,21 | 29,11±0,17 |
| 4 | 16,82±0,19 | 25,89±0,26 | 30,21±0,12 |
| 8 | 9,65±0,14 | 11,23±0,17 | 18,25±0,15 |
| 12 | 5,91±0,12 | 8,58±0,21 | 12,94±0,17 |
| 24 | 0 | 4,32±0,11 | 6,79±0,14 |
| P values vs. Control | | <0.01 | <0.001 |
| P values between CUREmin-ACTIF^{®} vs Test Sample | | | <0.001 |

In the second experimental set-up serum concentrations of curcumin II (desmethoxycurcumin) was determined. The results are summarized in Table II and graphically represented in Figure 4. Again the results show that the curcurmin II serum concentration of the Test Sample 3 according to the invention are higher compared to CUREmin-ACTIF^{®} 2 and Control 1. Those results lead to the assumption that the pharmaceutical composition according to the invention increases the bioavailability of curcumin.

**Table II**

| **Time (h)** | **Control (n=10) (ng/mL) Mean +/- SD** | **CUREmin-ACTIF^{®} (n=10) (ng/mL) Mean +/- SD** | **Test Sample (n=10) (ng/mL) Mean +/- SD** |
|---|---|---|---|
| **0** | **0** | **0** | **0** |
| 0,15 | 0,54±0,15 | 0,87±0,19 | 0,98±0,12 |
| 0,3 | 1,34±0,12 | 1,78±0,13 | 2,13±0,18 |
| 1 | 0,98±0,13 | 1,11±0,14 | 1,48±0,16 |
| 2 | 0,84±0.09 | 0,98±0,11 | 1,05±0,14 |
| 4 | 0,80±0,07 | 0,89±0,08 | 0,98±0,11 |
| 8 | 0,56±0.08 | 0,68±0,06 | 0,75±0,09 |
| 12 | 0 | 0,55±0,05 | 0,65±0,06 |
| 24 | 0 | 0 | 0 |
| P values vs. Control | | <0.01 | <0.005 |
| P value between CUREmin-ACTIF^{®} vs Test Sample | | | <0.05 |

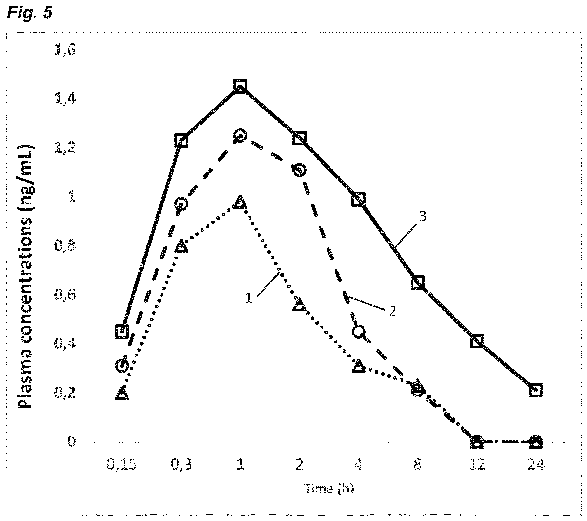

In the last experimental set-up the serum concentrations of curcumin III (bisdesmethoxycurcumin) in rats were determined in order to evaluate the bioavailability of the Test Sample 3 compared to CUREmin-ACTIF^{®} 2. The results are summarized in Table III and graphically represented in Figure 5. Again the results show that the curcurmin III serum concentration is higher after oral uptake of the Test Sample 3 than after oral uptake either of CUREmin-ACTIF^{®} 2 or Control 1. Furthermore the results even show that curcurmin III serum concentration could still be detected 24 hours after oral uptake of the Test Sample 3, whereas in case of CUREmin-ACTIF^{®} 2 or Control 1 the detection of curcumin III plasma concentration was no more possible 12 hours after oral uptake. Thus the bioavailability of curcumin is increased due to the pharmaceutical composition according to the invention.

**Table III**

| **Time (h)** | **Control (n=10) (ng/mL) Mean +/- SD** | **CUREmin-ACTIF^{®} (n=10) (ng/mL) Mean +/- SD** | **Test Sample (n=10) (ng/mL) Mean +/- SD** |
|---|---|---|---|
| **0** | **0** | **0** | **0** |
| 0,15 | 0,20±0,04 | 0,31±0,12 | 0,45±0,12 |
| 0,3 | 0,80±0,05 | 0,97±0,19 | 1,23±0,16 |
| 1 | 0,98±0,11 | 1,25±0,18 | 1,45±0,15 |
| 2 | 0,56±0,12 | 1,11±0,13 | 1,24±0,10 |
| 4 | 0,31±0,14 | 0,45±0,09 | 0,99±0,08 |
| 8 | 0,23±0,13 | 0,21±0,07 | 0,65±0,06 |
| 12 | 0 | 0 | 0,41±0,04 |
| 24 | 0 | 0 | 0,21±0.03 |
| P values vs. Control | | <0.05 | <0.0005 |
| P values between CUREmin-ACTIF^{®} vs Test Sample | | | <0.005 |

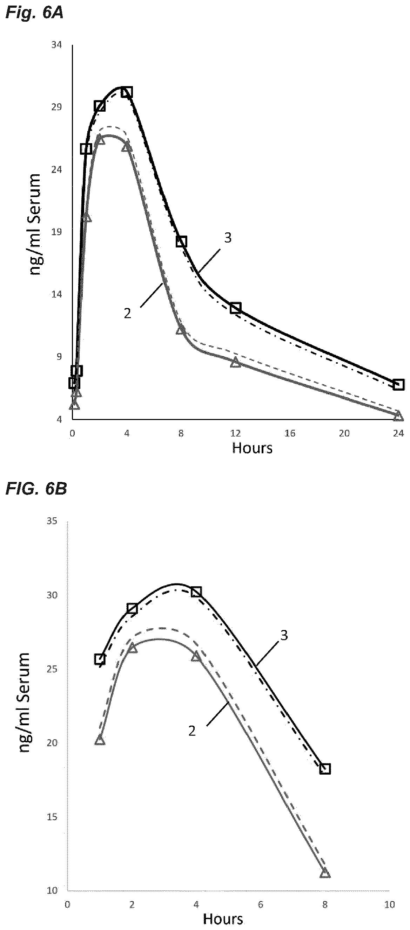

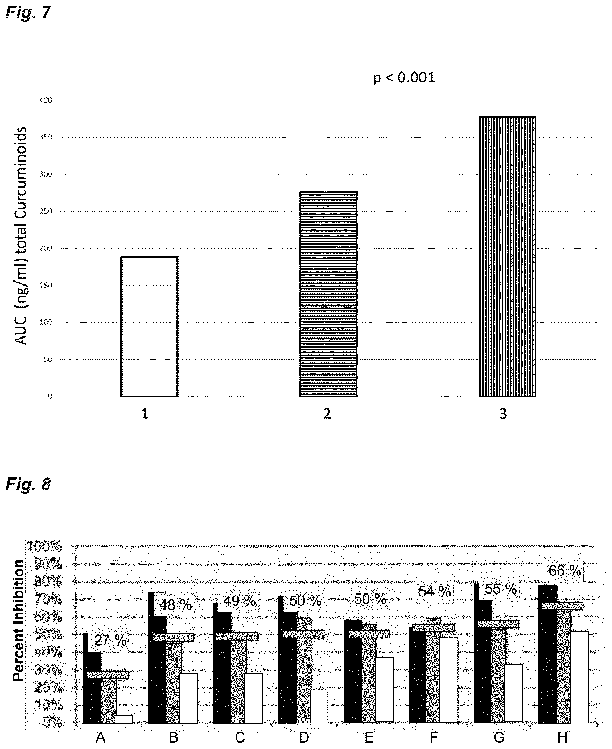

Furthermore, the pharmacokinetics of CUREmin-ACTIF^{®} 2 and the Test Sample 3 were compared. The results are represented in Figures 6A and 6B. The solid lines represent the calculated means of the concentrations in 10 rats, the dotted lines represent the standard deviation. The difference in achieved concentrations in serum between the two preparations are for the whole time-course of the experiment significantly different. Figure 6A shows the measured concentrations over the full time course of 0 - 24 hours, Figure 6B shows the concentrations for the time segment 1 to 8 hours. The concentrations of the Test Sample 3, which is pharmaceutical composition according to present invention, reached in serum exceed the TCD concentrations used here and depicted in Figure 8 - 10, for a time window of up to 8 hours by 3 to 10 fold. This shows, even if human and rat pharmacokinetics cannot be compared directly, that the inventive pharmaceutical composition can reach therapeutic concentrations *in vivo.* Figure 6B in additions shows, that the Tmax of Test Sample 3 is compared with CUREmin-ACTIF^{®} 2 shifted significantly by two hours (from 2 to 4 hours) to the right, indicating a longer half-life of the inventive pharmaceutical composition. Further, the Test Sample 3 shows at all times a higher bioavailability compared to CUREmin-ACTIF^{®} 2 (p<0,00001).

In Figure 7, the mean AUC's (Area under the curve) of the Control, unformulated curcuminoid, CUREmin-ACTIF^{®} 2 the Test Sample 3 are depicted. The Test Sample 3 achieves a significant higher serum concentration and availability as compared to CUREmin-ACTIF^{®} 2, which has been reported by Löffler, B.-M. et al in Food Chemistry and Analysis No. 25, Chapter 5, pages 104-135; 2021, to be so far the most active oral curcuminoid preparation.

### Example 5

Determination of the effect of a pharmaceutical composition according to the present invention, on human circulating tumor cells in blood of nine patients suffering from different types of carcinoma as given in the following Table IV. The composition of the pharmaceutical composition is the same as used in Example 4.

**Table IV**

| **Patient Number** | **Gender** | **diagnosis** |
|---|---|---|
| 1 | Male | Prostata Carcinoma |
| 2 | Male | Rectum Carcinoma |
| 3 | Female | Mamma carcinoma |
| 4 | Male | Lung carcinoma NET |
| 4b | Male | Lung carcinoma NET |
| 5 | Female | Metastasized |
| 6 | Female | Melanoma |
| 7 | Female | Triple negative |
| 8 | Korte | - |
| 9 | Male | glioblastom |

The cells from the collected blood were cultivated in a usual manner with an agent. The agent was either the pharmaceutical composition according to the present invention, or Resveratrol-ACTIF^{®}, which is a combination preparation comprising resveratrol and at least one gingerol, that were added to the cell culture in a concentration of either 50% 100% and 200%.

After a short incubation time the cell death was determined in percentage, which is shown in Table V. The results show that the pharmaceutical composition according to the present invention (Test Sample) showed in comparable concentrations a similar or higher efficacy as Resveratrol-ACTIF^{®}. Furthermore, those results show that the positive effects are dependent on the type of carcinoma.

**Table V**

| **Patient** | **Resveratrol-ACTIF^{®}** | | | **Test Sample** | | |
|---|---|---|---|---|---|---|
| | 50% | 100% | 200% | 50% | 100% | 200% |
| | Precent Cell Death | | | | | |
| 1 | 78 | 85 | 88 | 35 | 68 | 82 |
| 2 | 4 | 37 | 42 | 68 | 77 | 82 |
| 3 | 8 | 31 | 41 | 49 | 59 | 68 |
| 4 | 39 | 50 | 59 | 55 | 81 | 32 |
| 4b | 2 | 74 | 86 | 66 | 73 | 81 |
| 5 | 72 | 87 | 89 | 60 | 73 | 80 |
| 6 | 59 | 69 | 5 | nd | nd | nd |
| 7 | 72 | 80 | 80 | 78 | 80 | 80 |
| 8 | 21 | 45 | 58 | 20 | 45 | 58 |
| 9 | 79 | 88 | 90 | 65 | 70 | 82 |

Using a modified test system for the detection of tumor specific gene expression by qPCR as a measure of the presence of CTC (circulating tumor cells) the blood sample of patients was analysed. Circulating epithelial cells were enriched by immunomagnetic separation and selected tumor specific genes and separate housekeeping genes were quantified by qRT-PCR. Based on four positively detected epithelial CTC markers the CTC status was defined as positive.

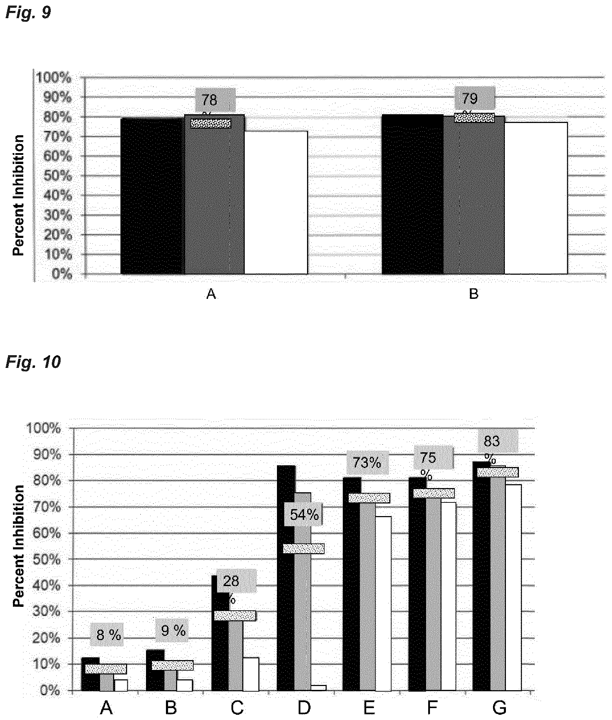

In Table VI three repetitive analysis of patient 7 are given to show the CTC inhibitory effect. The results given in Table VI correlate with the results given in Figure 8 and 9, which are explained in detail further below.

**Table VI**

| **Rec. No.:** | **Date** | **Tumor-associated Marker * [mRNA Copy Number]** | | | | | | | |
|---|---|---|---|---|---|---|---|---|---|
| | | EPCAM | KRT19 | MMG1 | MUC1 | SPDEF | ERBB2 | TERT | CTC-Score |
| 75182 | 31/08/2022 | (-) | (-) | (-) | 2 | 3 | (-) | (-) | 5 |
| 75230 | 08/09/2022 | (-) | (-) | (-) | 14 | 4 | 5 | 2 | 25 |
| 76739 | 15/06/2023 | 5 | (-) | (-) | 8 | 4 | 1 | (-) | 18 |

The same EDTA blood sample used for the verification of the CTC burden was used for drug sensitivity testing.

After enrichment of mononuclear cells by density gradient centrifugation, CD45 depletion and erythrolysis the remaining cells were suspended in cell culture medium and incubated for 4 days with and without the tested compounds. Compounds were tested at three tissue culture doses (TCD), namely 50%, 100% and 200%.. The doses for compounds of complementary oncology were adapted from normalized cell culture *in-vitro* concentrations. The results are presented in Figure 8, showing the inhibitory effect of the following classical chemotherapeutic compounds: A, Irinotecan; B, Epirubicin + Taxo-there; C, Epirubicin + Taxol; D, Epirubicin; E, Taxotere; F, Carboplatin; G, Taxol, H, Methadon. The 3 columns per compound/compound-group show three different concentrations applied in the experiment: Black, 200% TCD, Grey, 100% TCD, White, 50% TCD. TCD is the peak-plasma-dose which is achieved in human patients during a chemotherapy after single application of the drug. The respective doses are given in Table VII. It can be seen that the response of tumor cell-inhibition is concentration dependent, indicative for a specific response except for Carboplatin, for which as it seems already with the 50% TCD the maximal response has been reached. From theses chemotherapeutics Methadon, which is still not generally accepted as a valid drug in oncological therapy showed the highest antitumoral capacity.

**Table VII**

| **Chemotherapeutic drug** | **TCD** | | |
|---|---|---|---|
| | **200%** | **100%** | **50%** |
| | µg/ml | µg/ml | µg/ml |
| **Carboplatin** | 31,6 | 15,8 | 7,9 |
| **Taxol** | 9,6 | 4,8 | 2,4 |
| **Everolimus** | 0,8 | 0,4 | 0,2 |
| **Epirubicin** | 0,6 | 0,3 | 0,15 |
| **Taxotere** | 7,2 | 3,6 | 1,8 |
| **Irinotecan (as SN38)** | 0,2 | 0,1 | 0,05 |
| **Methadon** | 30,0 | 15,0 | 7,5 |

The same experiment was performed with human circulating tumor cells, prepared from a tumor patient by using Resveratrol-ACTIF^{®} and the pharmaceutical composition according to the present invention. The results are given in Figure 9 in respect of patient 7. Already in the 50% TCD both compounds reached nearly maximal efficacy of nearly 80% inhibition, by that exceeding all in parallel tested conventional chemotherapeutics. It should be stated here, that it is rather difficult, to always determine in the forefront the right concentration ranges to be tested. Tumor patients are individuals, so are their respective tumor cells. The results vary from patient to patient and therefore have to be determined individually. The course of the so called school medicine is - as can be shown - not goal leading.

Concentrations of chemotherapeutics used in the CTC-Assay are summarized in Table VIII. 100% reflects the maximal plasma peak concentration in human plasma after a maximal chemotherapeutic single dose. 200% and 50% respectively the double- / half-maximal therapeutic dose used in human.

**Table VIII**

| **TCD-Dose** | **Test Sample** | **Resveratrol-ACTIF^{®}** | **Methadon** |
|---|---|---|---|
| | ug/ml | ug/ml | ug/ml |
| 200% | 7,0 | 11,2 | 30,0 |
| 100% | 3,5 | 5,6 | 15 |
| 50% | 1,75 | 2,8 | 7,5 |

### Example 8

Superior anti-inflammatory potential of the pharmaceutical composition according to the invention.

17 patients with different chronic inflammation indications received oral therapy with the pharmaceutical composition according to the invention. The pharmaceutical composition used is the same as used in Example 3, namely a capsule of 250 mg Curcuminoids, 50 mg Turmeric Extract and 100 mg Sodium Starch octenyl succinate, which was produced by using the method according to the present invention. The results of the individual measurements of immunological indicator parameter are summarized in Table IXI. The individual results are given both for transparency and to illustrate the large differences in the possible elevations of cytokines, such as in TNF-α, IL-6, IL-8, or IL-1b. The measurement of the different parameters was performed in the usual manner by using methods like latex immunoassay, sequential enzyme-linked solid-phase chemiluminescence assay, MRP8/14 or Elisa. The first measurement (1st) was performed before, the second (2nd) 3 months after the start of the treatment intervention with a pharmaceutical composition according to the invention. The abbreviation nd means not measured. In case the value at the second measurement was below the respective detection limit (<5), this value was set to 4.9 for statistical analysis. Patient No. 6 had nonclassical lung carcinoma histologically classified as canceroid, and patient 14 had NSCLC. RA, rheumatoid arthritis. Patient 7 was on continuous treatment with Embrel^{®} (maximum dose). T2D, type 2 diabetes, HVS, herpes simplex virus.

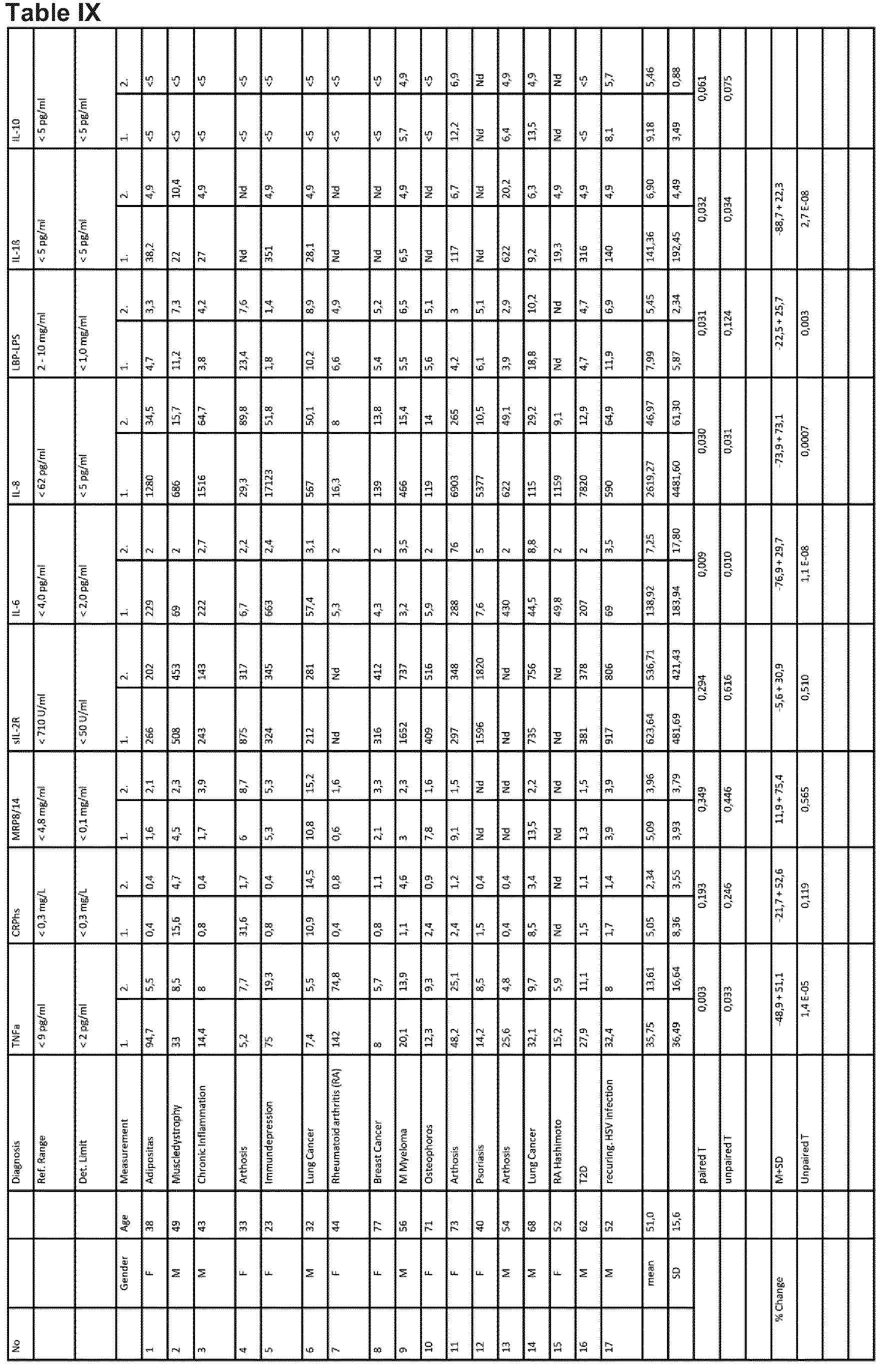

### Synergistic effects

The potential synergistic effects of resveratrol and curcumin can be explained by signal transduction pathway. Resveratrol inhibits AKT, mTOR, NPκB, MEK, ERK, β-Catenin, MITF, STAT3, c-kit, c-Jun, α-MSH; and curcumin inhibits Pl3K, AKT, NFκB, ERK, STAT3, whereas p53 is upregulated by resveratrol and curcumin. Additionally gingerol can have synergistic effects with resveratrol and curcumin as well. 6-gingerol has the following target structures in common with resveratrol: TNF-α, MMP 9/2, Akt, Cyclin D1, c-jun, JNK, AP-1, IL-1, NFkB, COX-2. Furthermore 6-gingerol regulates those in the same direction as resveratrol. 6-gingerol has the following target structures in common with curcumin: IL-1, TNF-a, JNK, MAPK, p53, COX-2, Cyclin D1, VEGF, EGF.

The synergistic potential of curcumin and resveratrol (in addition in comparison to standard chemotherapy) is shown in Fig. 10 in a case of a patient with a metastasized NET-lung-cancer: A, Everolimus; B, Taxol; C, Carboplatin; D, Resveratrol-ACTIF^{®}, E, pharmaceutical composition of the present invention; F, pharmaceutical composition of the present invention + Taxol; G, pharmaceutical composition of the present invention + Resveratrol-ACTIF^{®}.

The experimental set-up was similar to Example 5. Cells from a blood sample were cultivated with different agents. The agents were either chosen from classical chemotherapeutic compounds or alternative compounds such as resveratrol or curcumin as well as from pharmaceutical compositions according to the present invention. Examples for compositions according to the present invention are curcumin and resveratrol or curcumin, resveratrol and artesunat. As shown in the graph, with 50 % TCDs a combination of drugs improves the inhibitory effect to near maximum. Again, compositions according to the present invention have a higher effect than classical chemotherapeutic compounds.

Furthermore, the metabolic fate of resveratrol as well as curcumin at the gut wall is determined by enzymatic degradation. The involved enzymes (enzyme systems) SULT1A1, UGT1A1 und UGTA9 are not just active at the gut wall but also intrahepatically, which can be inhibited by gingerol.

The following table X shows the synergistic effects between curcumin (cur.), resveratrol (res.), 6-gingerol (6-ging.) and *Boswellia* (Bosw.).

**Table X**

| **Target** | **Cur. - 6- ging.** | **Cur. - Bosw.** | **Cur. - Res.** | **Bosw - Res.** | **Bosw. - 6-ging.** | **6-ging - Res.** |
|---|---|---|---|---|---|---|
| **Transcription factors** | NFkB | NFkB | NFkB | NFkB | NFkB | NFkB |
| | AP1 | STATS | AP1 | | | AP1 |
| **Kinases** | | | CREB-BP | | | C-Jun |
| | ERK 1/2 | ERK 1/2 | JNK1 | MAPK | ERK 1/2 | JNK |
| **Cytokines** | MAPK | | MAPK | | MAPK | |
| | TNF-a | | TNF-a | TNF-a | | TNF-a |
| | IL1 | | IL 1b | | | |
| | | | IL 6 | | | |
| | | | IL8 | | | |
| **Enzymes** | MMP 2 | MMP 9 | MMP 2 | MMP 9 | MMP 9 | COX 2 |
| | MMP 9 | COX 2 | MMP 9 | COX 2 | Caspase 9 | |
| | COX 2 | 5 LOX | COX 2 | | | |
| | | | GST-19 | | | |
| **Growth factors** | VEGF | VEGF | | | | |
| | | PDFG | | | | |
| | | TF | | | | |
| **Receptors** | | DR 5 | | | | |
| | | AR | | | | |
| | | CXCR | | | | |
| **Others** | P53 | P53 | VCAM | Cyclin D1 | | |
| | Cyclin D1 | Cyclin D1 | ICAM | | | |
| | | Suvivin | Cyclin D1 | | | |
| | | BCL-2 | | | | |
| | | BCL-xL | | | | |
| | | AP 1 | | | | |

### Reference Signs List

- 1: Control
- 2: CUREmin-ACTIF^{®}
- 3: Test Sample

- T: turmeric
- D: dried turmeric
- O: oleoresin
- C: curcuma extract or crystallized curcuma extract
- L: turmeric lipids
- S: starch or starch solution
- H: homogenised intermediate product
- M: double micro-encapsulated intermediate product
- P: pharmaceutical product

## Claims

1. A pharmaceutical composition, comprising a combination preparation comprising a first active ingredient derived from a plant oleoresin from plants of genus *curcuma* and at least one second active ingredient, double micro-encapsulated by a layer of carbohydrate polymers and lipids, wherein the lipids are derived from a plant oleoresin.

2. The pharmaceutical composition, according to claim 1, wherein at least the second active ingredient is resveratrol, gingerol or is derived from oleoresin from plants of genus *Boswellia .*

3. The pharmaceutical composition, according to claim 1 or 2, wherein the combination preparation comprises a third active ingredient, which is selected from resveratrol, gingerol or is derived from a plant oleoresin from plants of genus *Boswellia.*

4. The pharmaceutical composition, according to claim 1 or 2, wherein the lipids comprise a selection and/or a combination of fatty acids, glycerolipids, glycerophospholipids, sterols, prenols, saccharolipids and polyketides.

5. The pharmaceutical composition, according to any of the preceding claims, wherein the carbohydrate polymers are selected from one or more of the group comprising starch, modified starch, poly anhydrides, poly (ortho esters), poly (ethylene carbonates), poly (lactic acids), poly (glycol acids), poly (D,L-lactide-co-glycolide) and co-poly (L-lactic acid-oxyethylene-β-L-lactic acid).

6. The pharmaceutical composition, according to any of the preceding claims, wherein the at first active ingredient derived from plant oleoresin of genus *curcuma* comprises curcuminoids, especially curcumin, demethoxycurcumin, bis-demethoxycurcumin.

7. The pharmaceutical composition, according to any of the preceding claims, wherein the at least one second or third active ingredient derived from plant oleoresin of genus *Boswellia* comprises α-boswellic acid, acetyl-α-boswellic acid, acetyl-11-*keto*-α-boswellic acid, β-boswellic acid, acetyl-β-boswellic acid, 11-*keto*-β-boswellic acid (KBA), and/or acetyl-11-*keto*-β-boswellic acid (AKBA).

8. The pharmaceutical composition, according to any of the preceding claims, wherein the at least one second or third active ingredient of gingerol further comprises shogaols, which are selected from 4-shogaol, 6-shogaol, 8-shogaol, 10-shogaol and 12-shogaol.

9. The pharmaceutical composition, according to any of the preceding claims, wherein the double micro-encapsulated layer consists of an inner and an outer layer.

10. The pharmaceutical composition, according to claim 9, wherein the inner layer comprises a homogenized and/or complex mixture of a combination of the first active compound, namely a derivative from a plant oleoresin from plants of genus curcuma and a second active compound, namely resveratrol, gingerol or a derivative from plant oleoresin from plants of genus *Boswellia.*

11. The pharmaceutical composition, according to claim 9 or 10, wherein the outer layer comprises a homogenized and/or complex mixture of a combination of the first active compound, namely a derivative from a plant oleoresin from plants of genus curcuma and a second active compound, namely resveratrol, gingerol or a derivative from plant oleoresin from plants of genus *Boswellia.*

12. A method for the production of a double micro-encapsulated pharmaceutical composition, according to the claims 1 to 11, wherein the following steps are performed successively:
a. providing a plant oleoresin;
b. separating active ingredients in form of crystals from the plant oleoresin and separating lipids in form of liquids from the plant oleoresin;
c. mixing of the separated crystals and the separated lipids as obtained in step b. with carbohydrate polymers and forming an aqueous solution;
d. homogenization of the aqueous solution produced in step c.;
e. spray drying of in step d. produced homogenized aqueous solution and producing a double micro-encapsulated pharmaceutical composition.

13. The method, according to claim 12, wherein the providing of plant oleoresin according to step a. is performed in that a rhizome of the utilized plant of interest is dried and crushed and in that the oleoresin is extracted from the crushed rhizome material.

14. The method according to claim 13, wherein the extraction is performed using organic solvents comprising alcohols, like ethanol, methanol, and the like and/or acetone and/or hydrocarbons, like n-hexane, iso-hexane, n-pentane, iso-pentane, and the like and/or steam of water and/or supercritical fluid extraction using supercritical carbon dioxide.

15. The method according to claim 12, wherein the providing of plant oleoresin according to step a. is performed in that the oleoresin is extracted from excretion cells of the bark, especially from the plants of genus *Boswellia.*

16. The method according to claim 12 to 15, wherein in an additional step f. after step e. the product retrieved in step e., namely the double micro-encapsulated pharmaceutical composition is mixed with pharmaceutical additives or adjuvants and formed into a tablet or a capsule, wherein preferably the tablet or the capsule is an enteric coated tablet or an enteric coated capsule.

17. A pharmaceutical drug in form of an tablet or capsule, comprising a pharmaceutical composition according to any of claims 1 to 11.
